# EUROPEAN PATENT APPLICATION

(11) **EP 4 372 083 A1**
(43) Date of publication of application: **22.05.2024**
(21) Application number: 22842243.2
(22) Date of filing: 19.04.2022
(51) Int. Cl.: C12N 15/10

(54) **METHOD FOR EXTRACTING NUCLEIC ACID FROM POLYPHENOL-CONTAINING SAMPLE**

(30) Priority: 12.07.2021 KR 20210090890
(71) Applicant: Invirustech, Gwangju 61186 (KR)
(72) Inventor: PARK, Ki Beom, Gwangju 61120 (KR)
(74) Representative: Eisenführ Speiser
(86) International application number: PCT/KR2022/005577
(87) International publication number: WO 2023/286980

(57) **Abstract**

Disclosed are a method for extracting nucleic acids from a sample containing great amounts of polyphenols and/or polysaccharides, such as pears, bananas, and strawberries, and a buffer composition used therefor. The method of extracting nucleic acids from a sample includes mixing the sample with a first buffer containing Tris-HCl (tris(hydroxymethyl)aminomethane-HCl), mixing the sample with a second buffer containing sodium dodecyl sulfate (SDS), mixing the sample with a third buffer containing sodium chloride (NaCl), and mixing the sample with a fourth buffer containing guanidine hydrochloride (Gu-HCl).

## Description

### [Technical Field]

The present invention relates to a method for extracting nucleic acids from a sample. More specifically, the present invention relates to a method for extracting nucleic acids from a sample containing great amounts of polyphenols and/or polysaccharides and a buffer composition used therefor.

### [Background Art]

Nucleic acids refer to polymers including units called "nucleotides" and are a kind of biomolecules. Nucleic acids may be broadly classified into deoxyribonucleic acid (DNA) and ribonucleic acid (RNA).

The genetic information contained in nucleic acids is present in the base sequence of nucleic acids and is expressed in the form of proteins through biosynthesis. In addition, different organisms may have different combinations of DNA and RNA sequences since expressed proteins or enzymes play essential roles in the growth, maintenance, and division of cells. In addition, gene expression patterns of even identical organisms may be varied depending on external environmental conditions, nutritional status, presence or absence of pathogen infection, developmental stage, tissue differentiation, growth status and the like.

The varied expression patterns of genes may be expressed as RNA expression levels, gene expression levels, gene expression patterns, RNA transcription levels, changes in RNA transcription levels, transcriptome profiles, or the like. For this reason, purifying RNA or DNA from samples is the first step to be basically performed to determine the principle of life and presence or absence of infection, and to test reagents for a specific species through molecular biological tools.

In general, purified DNA is used for research of functional genomics. A specific region of DNA, for example, purified DNA, may be obtained through COI barcode, ITS barcode, ribosomal RNA sequencing, or the like, and may be used for biological identification through 1:1 comparison with public databases such as GenBank. In addition, purified DNA is used as a key basic material for various molecular biology basic research such as genetic manipulation.

Purified RNA may be synthesized into complement DNA through reverse transcription and the DNA synthesized in this way is also called a "cDNA library". cDNA may be prepared by the 1^{st} RT-PCR method using only specific primers depending on the method and procedure, and the cDNA library may be constructed using oligo dT between 10 and 20 mer or a random sequence between 5 and 20 mer. A PCR process performed with the synthesized cDNA library is referred to as a "two-step RT-PCR process". Based on the DNA synthesized through this process, DNA with a specific sequence can be exponentially amplified through a polymerase chain reaction, which is a unique characteristic of taq polymerase, using taq polymerase and sequence specific DNA oligo. Quantitative real-time PCR and gene expression relative quantification for estimating the amount of gene expression by tracking the amount of amplified DNA in real time, have been developed and are widely used worldwide through this principle.

In addition, genetic diseases or infectious diseases, such as bacteria and viruses, can be diagnosed by comparing DNA amplified based on the principle of specific amplification of a specific sequence, which is a unique phenomenon of PCR, between infected or non-infected samples. As such, PCR is a basic and most essential research tool in modern biology, which is currently used in almost all processes of manipulating and testing genetic materials.

### [Disclosure]

### [Technical Problem]

(Patent Document 1) KR Patent No. 10-0486179 granted on 2005.05.03, entitled "CELL LYSIS COMPOSITION, METHOD AND KIT FOR ISOLATING AND PURIFING NUCLEIC ACIDS"
(Patent Document 2) US Patent No. 5,234,809, granted on 1993.08.10., entitled "Process for isolating nucleic acid"
(Patent Document 3) KR Patent No. 10-2094079, granted on 2020.03.27., entitled "Composition for extracting viral RNA using silica-coated magnetic beads and method for extracting viral RNA using the same"
(Non-patent Document 1) Aranda, P. S., LaJoie, D. M., & Jorcyk, C. L. (2012). Bleach gel: a simple agarose gel for analyzing RNA quality. Electrophoresis, 33(2), 366-369.

As described above, PCR is a very powerful tool, but DNA polymerase, which is a key element of PCR, and a reverse transcriptase, which synthesizes RNA into cDNA, are functional protein enzymes and thus are sensitive to inhibitors, which are factors that inhibit the enzymes. These inhibitors inhibit the activity of enzymes in a concentration-dependent manner and thus must be maintained at low concentrations in all processes for a successful PCR experiment.

Unless a special experiment is performed, the inhibitors are contained in the purified RNA product or DNA product immediately before the enzyme reaction, and diagnosis and experiment results are greatly dependent on the quality of the nucleic acid extracted from the first sample. Therefore, extracting high-quality nucleic acids is of utmost importance to performing an accurate PCR process.

A method for extracting nucleic acids is based on destroying the cell walls of a sample, eluting nucleic acids therein, and filtering out impurities other than nucleic acids. Reagents used for nucleic acid extraction may be broadly classified into groups mainly containing chaotropic salts and groups containing phenol, chloroform, and the like.

Chaotropic salt-based products can extract high-quality RNA by inactivating RNA degrading enzymes based on the principle by which proteins are modified by strongly removing moisture from protein molecules. On the other hand, phenol-based products can extract high-quality RNA by separating RNA from proteins using protein solubility, and separating an RNA layer and a protein layer using chloroform.

Currently, a nucleic acid extraction kit from Qiagen Inc. is widely used. The nucleic acid extraction kit from Qiagen Inc. is a universal kit that can be primarily applied to unspecific samples at a laboratory level.

However, as described above, it is very important to extract high-quality nucleic acids in order to increase the reliability and accuracy of test results and reduce the manpower and time required for the test, in industrial fields that require high-level molecular diagnostic test results, especially in the field of infectious disease diagnosis.

In particular, extraction efficiency of nucleic acids may be completely changed depending on whether the sample is an animal or a plant, and secondary metabolites present in high concentrations in leaves or tissues, even if the sample is the same plant.

For another example, even when nucleic acids are extracted by the same method using the same detection buffer (or reagent), high-quality nucleic acids may be extracted from one sample, but may not be extracted from another sample, or may not be extracted at all from other sample.

Meanwhile, plants are known to be exposed to and infected with over 800 viruses. In particular, since it is practically difficult to treat plants having viral infection, unlike animals, there is a problem in that the area where plants are virally infected should be closed. Meanwhile, the recent expansion of the export and import markets of food crops has brought about increasing spread of crop diseases from one region to another region. For this reason, governments of each country conduct meticulous quarantine on imports and exports to prevent the spread of damage caused by virus-infected crops, but the period required for quarantine diagnosis is not short due to the ambiguity of the diagnosis results.

Plants contain a variety of DNA and RNA extraction inhibitors at higher amounts compared to animals due to biological characteristics thereof. It is not easy to extract high-quality nucleic acids from plants.

For example, plant leaves are the most frequently used samples for diagnosing plant viruses because they are the main organs where viruses proliferate. At the same time, leaves of plants contain high concentrations of polyphenols and polysaccharides accumulated by the protective mechanism of plans since they are most exposed to light, temperature, and pests.

However, polyphenols and/or polysaccharides may act as inhibitors in the process of extracting nucleic acids, such as RNA or DNA. In particular, commercially available nucleic acid extraction kits generally use a high concentration of guanidine salt. However, buffer conditions formed by such chaotropic salts may promote the formation of binding molecules or insoluble complexes by reaction of polyphenols and polysaccharides with nucleic acids, reduce diagnostic sensitivity for viruses or significantly hinder the construction of cDNA libraries that can be used for gene expression research. As a result, the time and cost required for diagnosis may be increased and erroneous negative judgment or test results may be derived.

When this phenomenon occurs during the import and export quarantine process of crops traded in billions of Korean Won, incorrect negative results may cause all crops to be arbitrarily disposed due to virus infection in the importing country or exporting country, which is a problem that may cause economic damage to the country depending on the responsibility.

In general, it is difficult to extract and recover high-quality nucleic acids from samples rich in polyphenols and/or polysaccharides using well-known nucleic acid extraction buffers. Among plants, in particular, samples rich in polyphenols and/or polysaccharides include crops such as pears, bananas, strawberries, mangoes, grapes, tomatoes, peaches, *Arabidopsis thaliana,* and pineapples, and fruits, stems, roots and/or leaves thereof.

Therefore, it is one object of the present invention to provide a reagent or buffer for extracting high-quality nucleic acids from samples rich in polyphenols and/or polysaccharides.

It is another object of the present invention to provide a kit including the reagent or buffer for extracting high-quality nucleic acids from samples rich in polyphenols and/or polysaccharides.

The objects of the present invention are not limited to those described above. Other objects of the present invention will be clearly understood from the following description.

### [Technical Solution]

In accordance with one aspect of the present invention, the above and other objects can be accomplished by the provision of a method of extracting nucleic acids from a sample including the following steps of mixing the sample with a first buffer containing Tris-HCl (tris(hydroxymethyl)aminomethane-HCl), mixing the sample with a second buffer containing sodium dodecyl sulfate (SDS), mixing the sample with a third buffer containing sodium chloride (NaCl), and mixing the sample with a fourth buffer containing guanidine hydrochloride (Gu-HCl).

The steps may be performed sequentially.

The first buffer may contain 0.8M to 1.2M Tris-HCl and may further contain 20 mM to 30 mM ethylenediaminetetraacetic acid (EDTA), 0.3% (w/v) to 0.7% (w/v) of sodium metabisulfite, sodium sulfite, sodium acid sulfite, or sodium sulfate, and 1.8% (w/v) to 2.2% (w/v) of polyvinylpyrrolidone, polyvinylpolypyrrolidone, or polyethylene glycol.

The second buffer may contain 3% (w/v) to 8% (w/v) of SDS and may further contain 20 mM to 30 mM EDTA, 120 mM to 180 mM sodium citrate, and 15 mM to 25 mM Tris-HCl.

The third buffer may contain 4.2M to 4.8M sodium chloride and may further contain 80 mM to 120 mM potassium chloride, calcium chloride, ferric chloride, ferrous chloride, ammonium aluminum, or ammonium sulfate, and 1.8% (w/v) to 2.2% (w/v) of polyvinylpyrrolidone, polyvinylpolypyrrolidone, or polyethylene glycol.

The fourth buffer may contain 3.5M to 4.5M guanidine hydrochloride.

In addition, the fourth buffer may further contain 10 mM to 20 mM Tris-HCl.

A volume ratio of the first buffer to the second buffer may be 1:1.1 to 1:1.2.

A volume ratio of the first buffer to the third buffer may be 1:0.6 to 1:0.8.

A volume ratio of the first buffer to the fourth buffer may be 1:0.9 to 1:1.1.

The first buffer may be used in an amount of 350 µl to 450 µl.

The step of mixing the sample with the first buffer may include mixing the first buffer, the sample, and 40 µl to 60 µl of a reducing agent, and pulverizing the sample.

In this case, the reducing agent may contain β-mercaptoethanol, 0.3M to 0.7M TCEP-HCl, or 0.8M to 1.2M dithiothreitol (DTT).

In addition, a volume ratio of the first buffer to the reducing agent may be 1:0.08 to 1:0.15.

In some embodiments, the method may further include primary incubation of incubating the mixture at 55°C to 65°C for 2 to 8 minutes after mixing with the second buffer and before mixing with the third buffer, secondary incubation of incubating the mixture for 30 seconds to 5 minutes after mixing with the third buffer and before mixing with the fourth buffer, primary centrifugation of centrifuging the cultured product after the secondary incubation and before mixing with the fourth buffer, tertiary incubation including incubating the mixture after mixing with the fourth buffer, secondary centrifugation of centrifuging the cultured product, and mixing the supernatant obtained from the secondary centrifugation with isopropanol and loading the mixture onto a column.

The mixing with the fourth buffer may include mixing the supernatant obtained from the primary centrifugation with the fourth buffer.

In some embodiments, the method may further sequentially include primary washing of washing the column with a fifth buffer containing isopropanol and sodium chloride, secondary washing of washing the column with a sixth buffer containing ethanol, Tris-HCl, and guanidine hydrochloride, tertiary washing of washing the column with a seventh buffer containing ethanol and Tris-HCl, and recovering nucleic acids.

The sample may be derived from fruit crops including pears, bananas, strawberries, mangoes, grapes, tomatoes, peaches, *Arabidopsis thaliana,* or pineapples.

In accordance with another aspect of the present invention, provided is a kit for extracting nucleic acids, the kit containing a plurality of buffers including a first buffer, a second buffer, a third buffer, and a fourth buffer, wherein the kit is configured to mix the sample with the first buffer and a reducing agent, to pulverize the sample, to mix the sample with the second to fourth buffers, to mix the sample with isopropanol, to load the sample on a column, and to wash the sample with at least one wash buffer.

The kit may include at least one of a first buffer, a second buffer, a third buffer, or a fourth buffer. For example, the kit may include all, three, two, or one of the first to fourth buffers.

Other aspects and preferred embodiments of the invention are discussed infra.

### [Advantageous effects]

According to embodiments of the present invention, high-quality nucleic acids can be extracted from samples rich in polyphenols and/or polysaccharides, for example, plant samples, more specifically, from crops such as pears, bananas, strawberries, mangoes, grapes, tomatoes, peaches, *Arabidopsis thaliana* or pineapples.

The effects according to the embodiments of the present invention are not limited to those exemplified above and other various effects are incorporated in the present specification.

### [Description of Drawings]

The above and other objects, features and other advantages of the present invention will be more clearly understood from the following detailed description taken in conjunction with the accompanying drawings, in which:
FIGS. 1 and 2 are flowcharts illustrating a method for extracting nucleic acids according to an embodiment of the present invention;
FIG. 3 is an image showing the result of nucleic acid extraction and electrophoresis according to Example 2;
FIG. 4 is an image showing the result of nucleic acid extraction and electrophoresis according to Comparative Example 1-1;
FIG. 5 is an image showing the result of nucleic acid extraction and electrophoresis according to Comparative Example 1-2;
FIG. 6 is an image showing the result of nucleic acid extraction and electrophoresis according to Comparative Example 2;
FIG. 7 is an image showing the result of nucleic acid extraction and electrophoresis according to Comparative Example 3;
FIG. 8 is an image showing the result of nucleic acid extraction and electrophoresis according to Comparative Example 4;
FIG. 9 is an image showing the result of nucleic acid extraction and electrophoresis according to Comparative Example 5;
FIGS. 10 to 12 are images showing the purity of nucleic acids measured according to Experimental Example 1; and
FIGS. 13 to 19 are images showing amplification curves and melting curves according to Experimental Example 2.

### [Best Mode]

Advantages, features, and methods to accomplish the same according to the present invention will be clearly understood from the following preferred embodiments with reference to the attached drawings. However, the present invention is not limited to the embodiments and may be embodied in different forms. The embodiments are suggested only to completely inform those skilled in the art of the scope of the present invention. The present invention will be defined only by the claims. In other words, a variety of modifications to the embodiments of the present invention are possible. The following embodiments of the present invention should not be construed as limiting the scope of the present invention, and those skilled in the art will appreciate that all modifications, additions and substitutions are possible.

All of the terms (including technical and scientific terms) used herein may be understood as having meanings that can be commonly understood by those skilled in the art to which the present invention pertains. In addition, terms defined in commonly used dictionaries should not be interpreted ideally or excessively unless explicitly specifically defined.

The term "and/or" used herein includes a designated single item and any combination of one or more items. Singular forms are intended to include plural forms as well, unless the context clearly indicates otherwise. It will be further understood that the terms "comprises" and/or "comprising" used herein do not preclude the presence or addition of at least one element other than the mentioned element. Numerical ranges indicated using the term "to" indicate numerical ranges including the values set forth before and after the term as lower and upper limits, respectively. The term "about" or "approximately" means a value or numerical range within 20% of the value or numerical range set forth after the term.

As used herein, the term "buffer" or "reagent" refers to a substance added, mixed, or added in the process of extracting nucleic acids and is not limited to the meaning of a buffer solution. The terms "buffer" and "reagent" may be used interchangeably.

As used herein, the term "primer" refers to a nucleic acid sequence having a short free 3' hydroxyl group, which is capable of forming a base pair with a complementary template of a nucleic acid and of acting as an initiation point for copying template strands.

Hereinafter, the present invention will be described in detail.

### First buffer composition

The first buffer composition may neutralize an acidic or basic pH of the sample to a neutral pH of about 7.0 and inhibit the oxidation process of an antioxidant in the sample. For example, the first buffer composition may be a buffer designed to prevent ROS and quinone that are generated by oxidation of polyphenols, which are major antioxidants, or may be designed to prevent chemical reactions with nucleic acids by adsorbing polyphenols. In addition, the first buffer composition may function as a homogenization solution that prevents the temperature of a sample from rapidly rising during a homogenization process, such as bead beating, causing generation of high heat.

The first buffer composition contains Tris-HCl (Tris buffer solution) and may further contain ethylenediaminetetraacetic acid (EDTA), sodium metabisulfite, and polyvinyl pyrrolidone.

The concentration of Tris-HCl may be about 0.8M to 1.2M, or about 0.9M to 1.1M. Tris-HCl may have a pH of about 6.9 to about 7.1, or about 7.0.

When the content of Tris-HCl is excessively small, the buffering effect may be reduced and the pH of the homogenate may change rapidly due to acidic or basic substances derived from the sample during sample homogenization. In this case, the quality or yield of nucleic acid, an extraction target, may decrease rapidly. On the other hand, when the content of Tris-HCl is excessively great, the osmotic pressure may increase and great amounts of endogenous RNase and DNase may be released in an inappropriate step, which may decrease the yield of nucleic acid.

In addition, the concentration of EDTA may be about 20 mM to about 30 mM, or about 22 mM to about 28 mM, or about 24 mM to about 26 mM.

When the content of EDTA is excessively small, DNA extraction efficiency may be lowered because the activity of DNase is not reduced, and cell walls may not be destroyed smoothly because calcium ions or other iron ions cannot be removed. On the other hand, when the content of EDTA is excessively great, the pH of the composition may increase and the desired pH buffering effect may be lost.

In some embodiments, the molar ratio of Tris-HCl to EDTA is about 35:1 to about 45:1, or about 36:1 to about 44:1, or about 37:1 to 43:1, or about 38:1 to about 42:1, or about 39:1 to about 41:1, or about 40:1.

The concentration of sodium metabisulfite may be about 0.3% (w/v) to about 0.7% (w/v), or about 0.4% (w/v) to about 0.6% (w/v), or about 0.5% (w/v) to about 0.6% (w/v). Here, % concentration in solution means % (w/v, weight/volume) unless otherwise defined.

When the content of sodium metabisulfite is excessively small, the oxidation of polyphenols cannot be prevented, and RNA and DNA extraction efficiency may be lowered.

In other embodiments, the first buffer composition may further contain sodium sulfite, sodium acid sulfite and/or sodium sulfate, instead of or in addition to sodium metabisulfite. In this case, the sum of the contents of sodium metabisulfite, sodium sulfite, sodium acid sulfite and sodium sulfate may be within the aforementioned concentration.

The concentration of polyvinylpyrrolidone may be about 1.8% (w/v) to about 2.2% (w/v), or about 1.9% (w/v) to about 2.1% (w/v), or about 2.0% (w/v). In another embodiment, instead of or in addition to polyvinylpyrrolidone, the first buffer composition may further contain polyvinylpolypyrrolidone or polyethylene glycol. In this case, the sum of the contents of polyvinylpyrrolidone, polyvinylpolypyrrolidone and polyethylene glycol may be within the aforementioned concentration.

When the content of polyvinylpyrrolidone (or polyvinylpolypyrrolidone, or polyethylene glycol) is excessively small, polyphenol cannot be effectively removed in the subsequent precipitation step due to the reduced amount of adsorbed polyphenol. In this case, polyphenols react with nucleic acids, thus decreasing the extraction efficiency of RNA and DNA. When the content is excessively great, the viscosity of the solution may increase, causing difficulty in homogenization and an increase in difficulty in the subsequent liquid handling process.

In some embodiments, the weight ratio of sodium metabisulfite to polyvinylpyrrolidone or the like may be about 1:3.8 to about 1:4.2, or about 1:3.9 to about 1:4.1, or about 1:4.

In an exemplary embodiment, the first buffer composition may contain a cationic surfactant such as a guanidine salt, phenol, chloroform, iso-amyl alcohol, and/or cetyl trimethylammonium bromide (CTAB).

As will be described later, the first buffer composition may be a buffer that is first mixed with the pulverized sample rich in polyphenols and/or polysaccharides. In this case, the first buffer composition according to the present embodiment may not contain a guanidine salt, thus preventing a chemical reaction between polyphenol and polysaccharide.

In addition, the first buffer composition is free from a substance that is highly volatile and harmful to the human body, such as phenol, chloroform and/or isoamyl alcohol, thus promoting the safety of operators extracting nucleic acids. Also, the first buffer composition enables stable operation under relatively mild conditions without a complicated equipment, thus realizing operation with a kit for on-site diagnosis. In addition, the first buffer composition does not contain cetyltrimethylammonium bromide, thus eliminating the necessity of the use of liquid nitrogen and ethanol co-precipitation. Therefore, when liquid nitrogen or ethanol co-precipitation is not selectively used depending on the sample, it is possible to simplify the nucleic acid extraction process, prevent the sample from being contaminated in the process of pulverizing and homogenizing the sample using liquid nitrogen, and shorten the time required for nucleic acid extraction.

### Method for preparing first buffer composition

The first buffer composition may be prepared by mixing Tris-HCl and further mixing EDTA, sodium metabisulfite, and polyvinylpyrrolidone or polyethylene glycol. The mole (mol), volume (ml), and weight (g)-based content and concentration of each component, and replaceable materials thereof are as described above.

In an exemplary embodiment, the first buffer composition may be prepared by mixing 25 ml of 2M Tris-HCl (pH 7.0), 2.5 ml of 0.5M EDTA, 0.25 g of sodium metabisulfite (powder) and 1.0 g of polyvinylpyrrolidone (pH of 3.0 to 5.0, molar average molecular weight of 25,000 to 35,000, powder). In this case, the volume of the prepared first buffer composition may be about 50 ml.

However, the present invention is not limited thereto and when the mole (mol), volume (ml) and weight (g)-based contents are derived, the weight and volume of each mixed component are not limited. In addition, the molarity (M) of each component may be the same as that of a commercially available product or may be changed by dilution.

The total amount (volume) of the first buffer composition may be about 100 ml or less, or about 90 ml or less, or about 80 ml or less, or about 70 ml or less, or about 60 ml or less, or about 50 ml or less, or about 40 ml or less, or about 30 ml or less.

The lower limit of the total amount of the first buffer composition is not particularly limited, but may be about 300 µl or more, or about 350 µl or more, or about 400 µl or more, or about 450 µl or more, or about 500 µl or more, or about 550 µl or more, or about 600 µl or more, or about 650 µl or more, or about 700 µl or more, or about 750 µl or more, or about 800 µl or more, or about 850 µl or more, or about 900 µl or more, or about 950 µl or more, or about 1,000 µl or more, in consideration of high-quality nucleic acid extraction.

### Second Buffer Composition

The second buffer composition contains a surfactant to destroy and modify cells and proteins, and to adsorb proteins. In addition, the second buffer composition may at least partially remove polysaccharides.

The second buffer composition contains sodium dodecyl sulfate (SDS) and may further contain EDTA, sodium citrate, and Tris-HCl (Tris buffer solution).

The concentration of SDS may be about 3% (w/v) to about 8% (w/v), or about 4% (w/v) to about 7% (w/v), or about 5% (w/v) to about 6% (w/v).

When the content of SDS is excessively small, the eluted RNA may be degraded due to the reduced denaturation of proteins, for example, the denaturation of RNase. The extraction efficiency of RNA and DNA may decrease because the efficiency of cell destruction may decrease and the amount of elution of RNA or DNA may decrease. When the content of SDS is excessively great, the difficulty of handling in subsequent steps may be increased due to excessive reaction of SDS with proteins or other compounds in the buffer composition, and low extraction efficiency may be reduced due to adsorption of RNA and DNA as well.

In addition, the concentration of EDTA may be about 20 mM to about 30 mM, or about 22 mM to about 28 mM, or about 24 mM to about 26 mM.

When the content of EDTA is excessively small, DNA extraction efficiency may be lowered because the activity of DNase is not reduced, and cell walls cannot be destroyed smoothly because calcium ions or other iron ions cannot be removed. When the content of EDTA is excessively great, the pH of the composition may increase and the desired pH buffering effect may be lost.

The concentration of sodium citrate may be about 120 mM to about 180 mM, or about 130 mM to about 170 mM, or about 140 mM to about 160 mM, or about 150 mM.

When the content of sodium citrate is excessively small, DNA extraction efficiency may be lowered because the activity of DNase is not reduced, and cell wall destruction cannot be achieved smoothly because calcium ions or other iron ions cannot be removed. When the content of sodium citrate is excessively high, the pH of the composition may increase and the desired pH buffering effect may be lost.

In some embodiments, the mole ratio of EDTA to sodium citrate may be about 1:4 to about 1:8, or about 1:5 to about 1:7, or about 1.6.

In addition, the concentration of Tris-HCl may be about 15 mM to about 25 mM, or about 16 mM to about 24 mM, or about 17 mM to about 23 mM, or about 18 mM to about 22 mM, or about 19 mM to about 21 mM. The pH of Tris-HCl may be about 7.4.

When the content of Tris-HCl is excessively small, the buffering effect is reduced, and the pH of the homogenate may change rapidly due to acidic or basic substances derived from the sample during sample homogenization. In this case, the quality or yield of nucleic acid as an extraction target may decrease rapidly. When the content of Tris-HCl is excessively great, the osmotic pressure may increase and large amounts of endogenous RNase and DNase may be released in an undesired step, which may decrease the yield of nucleic acid.

In some embodiments, the molar ratio of EDTA to Tris-HCl may be about 1:1 to about 1.5:1, or about 1.1:1 to about 1.4:1, or about 1.2:1 to about 1.3:1, or about 1.25:1.

### Method for preparing second buffer composition

The second buffer composition may be prepared by mixing SDS and further mixing EDTA, sodium metabisulfite, and Tris-HCl. The mole (mol), volume (ml), and weight (g)-based content and concentration of each component, and replaceable materials thereof are as described above.

In an exemplary embodiment, the second buffer composition may be prepared by mixing 12.5 ml of 20% SDS, 2.5 ml of 0.5M EDTA, 1.93 g of sodium citrate (powder), and 1.0 ml of 1.0M Tris-HCl (pH 7.4). In this case, the volume of the prepared second buffer composition may be about 50 ml.

However, the present invention is not limited thereto and, when the mole (mol), volume (ml) and weight (g)-based contents are derived, the weight and volume of each mixed component are not limited. In addition, the molarity (M) of each component may be the same as that of a commercially available product or may be changed by dilution.

The total amount (volume) of the second buffer composition may be about 100 ml or less, or about 90 ml or less, or about 80 ml or less, or about 70 ml or less, or about 60 ml or less, or about 50 ml or less, or about 40 ml or less, or about 30 ml or less.

The lower limit of the total amount of the second buffer composition is not particularly limited, but may be about 300 µl or more, or about 350 µl or more, or about 400 µl or more, or about 450 µl or more, or about 500 µl or more, or about 550 µl or more, or about 600 µl or more, or about 650 µl or more, or about 700 µl or more, or about 750 µl or more, or about 800 µl or more, or about 850 µl or more, or about 900 µl or more, or about 950 µl µl or more, or about 1,000 µl or more, in consideration of high-quality nucleic acid extraction.

### Third Buffer Composition

The third buffer composition may form a high osmotic pressure and neutralize the surface charge of the nucleic acid eluted due to the second buffer composition to reduce a solvent phase dissolution rate. In addition, the third buffer composition may remove the SDS of the second buffer composition by adsorption and/or precipitation, precipitate proteins and polysaccharides, and facilitate nucleic acid binding. In addition, the third buffer composition may prevent nucleases such as RNase and DNase from accessing since sodium ions react with phosphate of nucleic acids and thus coat the surface.

The third buffer composition contains sodium chloride (NaCl) and may further contain potassium chloride (KCl) and polyvinylpyrrolidone.

The concentration of sodium chloride may be about 4.2M to 4.8M, or about 4.3M to 4.7M, or about 4.4M to about 4.6M, or about 4.5M.

When the content of sodium chloride is excessively small, the osmotic pressure may be lowered and the effects of forming osmotic pressure, neutralizing nucleic acid surface charges, forming nucleic acid binding, inducing precipitation of proteins and polysaccharides, and inducing SDS adsorption and precipitation cannot be obtained. When the content of sodium chloride is excessively great, the solution may become saturated, and sodium chloride and other additives may precipitate out.

The concentration of potassium chloride may be about 80 mM to about 120 mM, or about 90 mM to about 110 mM. In other embodiments, the third buffer composition may further contain ferric chloride, ferrous chloride, aluminum ammonium and/or ammonium sulfate, instead of or in addition to potassium chloride. In this case, the sum of the contents of calcium chloride, ferric chloride, ferrous chloride, aluminum ammonium and ammonium sulfate may be within the concentration described later.

When the content of potassium chloride is excessively small, the stability of the precipitate may decrease because the bonding force between SDS and NaCl cannot be promoted. When the content of potassium chloride is excessively great, the nucleic acid may react with potassium and potassium may be eluted at the final recovery step, which may interfere with the PCR process or cause malfunction.

In some embodiments, the molar ratio of sodium chloride to potassium chloride or the like may be about 40:1 to about 50:1, or about 41:1 to about 49:1, or about 42:1 to about 48:1, or about 43:1 to about 47: 1, or about 44:1 to about 46:1, or about 45:1.

The concentration of polyvinylpyrrolidone may be about 1.8% (w/v) to about 2.2% (w/v), or about 1.9% (w/v) to about 2.1% (w/v), or about 2.0% (w/v). In another embodiment, instead of or in addition to polyvinylpyrrolidone, the third buffer composition may further contain polyvinylpolypyrrolidone or polyethylene glycol. In this case, the sum of the contents of polyvinylpyrrolidone, polyvinylpolypyrrolidone and polyethylene glycol may be within the aforementioned concentration.

When the content of polyvinylpyrrolidone (or polyvinylpolypyrrolidone, or polyethylene glycol) is excessively small, polyphenol cannot be effectively removed in the subsequent precipitation step due to the adsorption of reduced amounts of polyphenol. In this case, polyphenols may react with nucleic acids and the extraction efficiency of RNA and DNA may be lowered. When the content is excessively great, the viscosity of the solution increases, causing difficulty in homogenization and increasing the difficulty of the subsequent liquid handling process.

### Method for preparing third buffer composition

The third buffer composition may be prepared by mixing sodium chloride and further mixing potassium chloride and polyvinylpyrrolidone. The mole (mol), volume (ml), and weight (g)-based content and concentration of each component, and replaceable materials thereof are as described above.

In an exemplary embodiment, the third buffer composition may be prepared by mixing 45 ml of 5M sodium chloride, 0.37 g of potassium chloride (powder), and 1.0 g of polyvinylpyrrolidone (pH of 3.0 to 5.0, molar average molecular weight of 25,000 to 35,000, powder). In this case, the volume of the prepared third buffer composition may be about 50 ml.

However, the present invention is not limited thereto, and when the mole (mol), volume (ml) and weight (g)-based contents are derived, the weight and volume of each mixed component are not limited. In addition, the molarity (M) of each component may be the same as that of a commercially available product or may be changed by dilution.

The total amount (volume) of the third buffer composition may be about 100 ml or less, or about 90 ml or less, or about 80 ml or less, or about 70 ml or less, or about 60 ml or less, or about 50 ml or less, or about 40 ml or less, or about 30 ml or less.

The lower limit of the total amount of the third buffer composition is not particularly limited, but may be about 300 µl or more, or about 350 µl or more, or about 400 µl or more, or about 450 µl or more, or about 500 µl or more, or about 550 µl or more, or about 600 µl or more, or about 650 µl or more, or about 700 µl or more, or about 750 µl or more, or about 800 µl or more, or about 850 µl or more, or about 900 µl or more, or about 950 µl or more, or about 1,000 µl or more, in consideration of high-quality nucleic acid extraction.

### Fourth Buffer Composition

The fourth buffer composition may precipitate polyvinylpyrrolidone bound with SDS and polyphenols of the second buffer composition. In addition, the fourth buffer composition may denature remaining proteins and facilitate nucleic acid binding.

The fourth buffer composition contains guanidine-HCl, and may further contain Tris-HCl.

The concentration of guanidine-HCl may be about 3.5M to about 4.5M, or about 3.6M to about 4.4M, or about 3.7M to about 4.3M, or about 3.8M to about 4.2M, or about 3.9M to about 4.1M.

When the content of guanidine-HCl is excessively small, PVP bound to polyphenols cannot be precipitated, and as a result, the extracted nucleic acids may be contaminated with polyphenols. In addition, the efficiency of nucleic acid binding to the silica membrane may be reduced and thus the extraction efficiency of nucleic acids may be reduced. When the content of guanidine-HCl is excessively great, the solution may be saturated, and sodium chloride and other additives of the third buffer composition may be precipitated.

In addition, the concentration of Tris-HCl may be about 10 mM to about 20 mM, or about 12 mM to about 18 mM, or about 14 mM to about 16 mM. The pH of Tris-HCl may be about 7.4. The Tris-HCl content or concentration of the fourth buffer composition may be smaller than that of the second buffer composition.

When the content of Tris-HCl is excessively small, the buffering effect may be reduced, and when the content is excessively great, pH may increase rapidly or other additives may be precipitated.

In some embodiments, the molar ratio of guanidine-HCl to Tris-HCl may be 250:1 to 280:1, or about 260:1 to about 270:1, or about 265:1 to about 268:1, or about 266:1 to about 267:1.

### Method for preparing fourth buffer composition

The fourth buffer composition may be prepared by mixing guanidine-HCl and further mixing Tris-HCl. The mole (mol), volume (ml), and weight (g)-based content and concentration of each component, and replaceable materials thereof are as described above.

In an exemplary embodiment, the fourth buffer composition may be prepared by mixing 19.1 g of guanidine hydrochloride (powder) and 0.75 ml of 1.0M Tris-HCl (pH 7.4) . In this case, the volume of the prepared fourth buffer composition may be about 50 ml.

However, the present invention is not limited thereto, and when the mole (mol), volume (ml) and weight (g)-based contents are derived, the weight and volume of each mixed component are not limited. In addition, the molarity (M) of each component may be the same as that of a commercially available product or may be changed by dilution.

The total amount (volume) of the fourth buffer composition may be about 100 ml or less, or about 90 ml or less, or about 80 ml or less, or about 70 ml or less, or about 60 ml or less, or about 50 ml or less, or about 40 ml or less, or about 30 ml or less.

The lower limit of the total amount of the fourth buffer composition is not particularly limited, but may be about 300 µl or more, or about 350 µl or more, or about 400 µl or more, or about 450 µl or more, or about 500 µl or more, or about 550 µl or more, or about 600 µl or more, or about 650 µl or more, or about 700 µl or more, or about 750 µl or more, or about 800 µl or more, or about 850 µl or more, or about 900 µl or more, or about 950 µl or more, or about 1,000 µl or more, in consideration of high-quality nucleic acid extraction.

### Fifth Buffer Composition

The fifth buffer composition may be a first wash buffer used after loading into the column. The fifth buffer composition may contain isopropanol, sodium chloride, and a nonionic surfactant. In particular, the fifth buffer composition may aim at removing peculiar pigments such as chlorophyll contained in a large amount in plant tissues.

The concentration of isopropanol may be about 55% (v/v, volume/volume) and 65% (v/v), or about 60% (v/v).

When the content of isopropanol is excessively small, the nucleic acid does not precipitate out of the lysate, making it difficult to bind to a silica membrane or the like, and when the content of isopropanol is excessively great, the volume of the entire mixture increases, thus causing increased difficulty of handling, precipitation of substances other than nucleic acids, and contamination of nucleic acids.

In addition, the concentration of sodium chloride may be about 120 mM to about 180 mM, or about 130 mM to about 170 mM, or about 140 mM to about 160 mM.

When the content of sodium chloride is excessively small, the binding force between the solid support such as silica membrane and nucleic acid decreases during washing, and nucleic acids may be lost in the washing process, and when the content of sodium chloride is excessively great, sodium chloride may be precipitated and the cleaning efficiency may be reduced, such as an increase in the degree of contamination of sodium chloride.

The concentration of the nonionic surfactant is about 0.1% (v/v) to about 0.3% (v/v), or about 0.15% (v/v) to about 0.25% (v/v), or about 0.2% (v/v).

When the concentration of the nonionic surfactant is excessively small, the contact area between a solid support such as a silica membrane and a washing buffer may decrease, and washing efficiency may decrease due to the hydrophobicity of the silica membrane itself. When the concentration of the nonionic surfactant is excessively great, bubbles may be generated during the process, thus increasing the difficulty of handling, and the possibility of sample-to-sample contamination by particles scattered when the generated bubbles are destroyed.

### Method for preparing fifth buffer composition

The fifth buffer composition may be prepared by mixing isopropanol, sodium chloride, and a nonionic surfactant. The mole (mol), volume (ml), and weight (g)-based content and concentration of each component, and replaceable materials thereof are as described above.

In an exemplary embodiment, the fifth buffer composition may be prepared by mixing 30 ml of 99% isopropanol, 1.5 ml of 5M sodium chloride, and 10 µl of 99% tween-21. In this case, the volume of the prepared fifth buffer composition may be about 50 ml.

However, the present invention is not limited thereto, and when the mole (mol), volume (ml) and weight (g)-based contents are derived, the weight and volume of each mixed component are not limited. In addition, the molarity (M) of each component may be the same as that of a commercially available product or may be changed by dilution.

The total amount (volume) of the fifth buffer composition may be about 100 ml or less, or about 90 ml or less, or about 80 ml or less, or about 70 ml or less, or about 60 ml or less, or about 50 ml or less, or about 40 ml or less, or about 30 ml or less. The lower limit of the total amount of the fifth buffer composition is not particularly limited, but may be about 300 µl or more, or about 350 µl or more, or about 400 µl or more, or about 450 µl or more, or about 500 µl or more, or about 550 µl or more, or about 600 µl or more, or about 650 µl or more, or about 700 µl or more, or about 750 µl or more, or about 800 µl or more, or about 850 µl or more, or about 900 µl or more, or about 950 µl or more, or about 1,000 µl or more, in consideration of high-quality nucleic acid extraction.

### Sixth Buffer Composition

The sixth buffer composition may be a secondary wash buffer. The sixth buffer composition may contain ethanol, guanidine hydrochloride (Gu-HCl), and Tris-HCl. In particular, guanidine hydrochloride may be used to cause denaturation of proteins and remove the proteins from a solid support such as a silica membrane.

The concentration of ethanol may be about 55% (v/v, volume/volume) to about 65% (v/v), or about 60% (v/v).

When the content of ethanol is excessively small, nucleic acids may be dissolved in the buffer composition and may be lost during the washing process, and when the content of ethanol is excessively great, guanidine hydrochloride, which is an additive, may be precipitated and the desired protein removal efficiency may be reduced.

In addition, the concentration of guanidine hydrochloride may be about 1.5M to about 2.0M, or about 1.6M to about 1.8M, or about 1.65M to about 1.7M.

When the content of guanidine hydrochloride is excessively small, protein removal cannot be performed smoothly due to reduced protein denaturation efficiency and the quality and yield of nucleic acid may be reduced because nucleic acid is eluted during the washing process. When the content of guanidine hydrochloride is excessively great, guanidine hydrochloride is not removed in the subsequent washing process, which may interfere with analysis processes such as PCR and RT-PCR, which are subsequent processes after nucleic acid extraction.

The concentration of Tris-HCl may be about 10 mM to about 20 mM, or about 12 mM to about 18 mM, or about 14 mM to about 16 mM. The pH of Tris-HCl may be about 7.4. The Tris-HCl content or concentration of the sixth buffer composition may be smaller than that of the second buffer composition.

When the content of Tris-HCl is excessively small, the buffering effect may be reduced, and when the content is excessively great, pH may increase rapidly or other additives may precipitate.

### Method for preparing sixth buffer composition

The sixth buffer composition may be prepared by mixing ethanol, guanidine-HCl and Tris-HCl. The mole (mol), volume (ml), and weight (g)-based content and concentration of each component, and replaceable materials thereof are as described above.

In an exemplary embodiment, the sixth buffer composition may be prepared by mixing 30 ml of 99% ethanol, 7.98g of 1.67M guanidine hydrochloride (powder) and 0.75 ml of 1.0M Tris-HCl (pH 7.4). In this case, the volume of the prepared sixth buffer composition may be about 50 ml.

However, the present invention is not limited thereto, and when the mole (mol), volume (ml) and weight (g)-based contents are derived, the weight and volume of each mixed component are not limited. In addition, the molarity (M) of each component may be the same as that of a commercially available product or may be changed by dilution.

The total amount (volume) of the sixth buffer composition may be about 100 ml or less, or about 90 ml or less, or about 80 ml or less, or about 70 ml or less, or about 60 ml or less, or about 50 ml or less, or about 40 ml or less, or about 30 ml or less. The lower limit of the total amount of the sixth buffer composition is not particularly limited, but may be about 300 µl or more, or about 350 µl or more, or about 400 µl or more, or about 450 µl or more, or about 500 µl or more, or about 550 µl or more, or about 600 µl or more, or about 650 µl or more, or about 700 µl or more, or about 750 µl or more, or about 800 µl or more, or about 850 µl or more, or about 900 µl or more, or about 950 µl or more, or about 1,000 µl or more, in consideration of high-quality nucleic acid extraction.

### Seventh Buffer Composition

The seventh buffer composition may be a tertiary wash buffer. The seventh buffer composition may contain ethanol, sodium chloride, and Tris-HCl. In particular, the seventh buffer composition may be used to remove the high-concentration salt of the composition and remove the remaining silica membrane and pigments remaining on the solid support.

The concentration of ethanol may be about 70% (v/v) to about 80% (v/v), or about 71% (v/v) to about 79% (v/v), or about 72% (v/v) to about 78% (v/v), or about 73% (v/v) to about 77% (v/v), or about 74%(v/v) to about 76% (v/v).

When the content of ethanol is excessively small, nucleic acids may be dissolved in the buffer composition and may be lost during the washing process, and when the content of ethanol is excessively great, remaining salts may not be dissolved and may be eluted as well in the final extraction step.

The concentration of sodium chloride may be about 15 mM to about 25 mM, or about 16 mM to about 24 mM, or about 17 mM to about 23 mM, or about 18 mM to about 22 mM, or about 19 mM to about 21 mM.

When the content of sodium chloride is excessively small, the binding force between the solid support such as silica membrane and nucleic acid decreases during washing, and nucleic acids may be lost in the washing process. When the content of sodium chloride is excessively great, sodium chloride may precipitate and the cleaning efficiency may be reduced, such as an increase in the degree of contamination of sodium chloride.

The concentration of Tris-HCl may be about 10 mM to about 20 mM, or about 12 mM to about 18 mM, or about 14 mM to about 16 mM. The pH of Tris-HCl may be about 7.4. The Tris-HCl content or concentration of the sixth buffer composition may be smaller than that of the second buffer composition.

When the content of Tris-HCl is excessively small, the buffering effect may be reduced, and when the content is excessively great, pH may increase rapidly or other additives may be precipitated.

### Method for preparing seventh buffer composition

The seventh buffer composition may be prepared by mixing ethanol, sodium chloride, and Tris-HCl. The mole (mol), volume (ml), and weight (g)-based content and concentration of each component, and replaceable materials thereof are as described above.

In an exemplary embodiment, the seventh buffer composition may be prepared by mixing 37.5 ml of 99% ethanol, 0.2 ml of 5M sodium chloride and 0.75 ml of 1M Tris-HCl (pH 7.4) . In this case, the volume of the prepared seventh buffer composition may be about 50 ml.

However, the present invention is not limited thereto, and when the mole (mol), volume (ml) and weight (g)-based contents are derived, the weight and volume of each mixed component are not limited. In addition, the molarity (M) of each component may be the same as that of a commercially available product or may be changed by dilution.

The total amount (volume) of the seventh buffer composition may be about 100 ml or less, or about 90 ml or less, or about 80 ml or less, or about 70 ml or less, or about 60 ml or less, or about 50 ml or less, or about 40 ml or less, or about 30 ml or less. The lower limit of the total amount of the sixth buffer composition is not particularly limited, but may be about 300 µl or more, or about 350 µl or more, or about 400 µl or more, or about 450 µl or more, or about 500 µl or more, or about 550 µl or more, or about 600 µl or more, or about 650 µl or more, or about 700 µl or more, or about 750 µl or more, or about 800 µl or more, or about 850 µl or more, or about 900 µl or more, or about 950 µl or more, or about 1,000 µl or more, in consideration of high-quality nucleic acid extraction.

### Nucleic acid extraction method

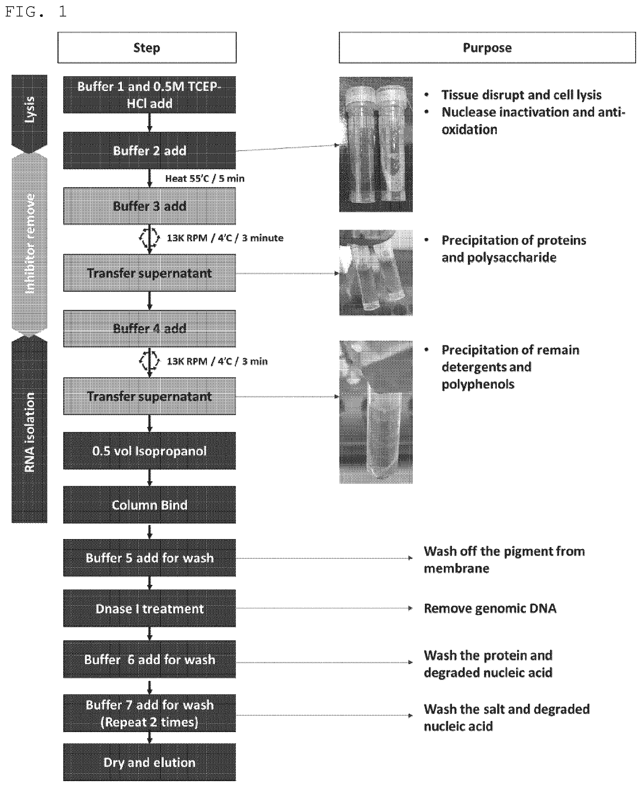

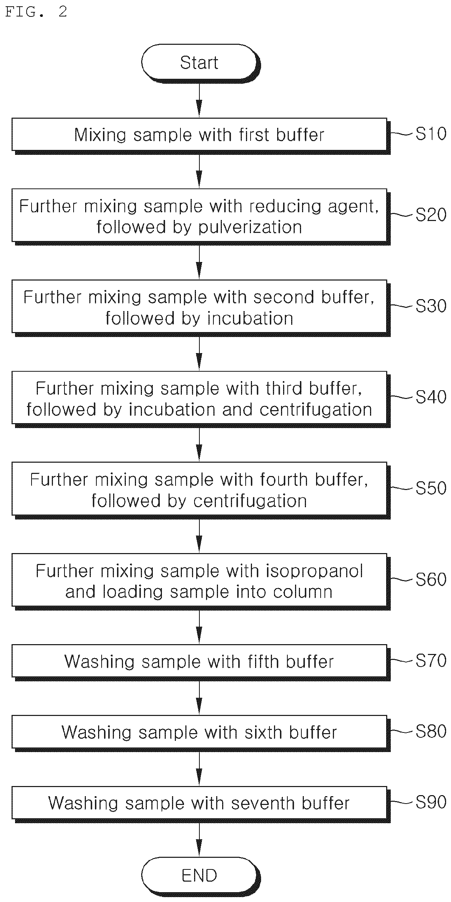

FIGS. 1 and 2 are flowcharts illustrating a method for extracting nucleic acids according to an embodiment of the present invention.

The nucleic acid extraction method according to the present invention includes mixing a sample with a first buffer (S10), further mixing the resulting sample with a reducing agent, followed by pulverization (S20), further mixing the sample with a second buffer, followed by incubation (S30), further mixing the resulting sample with a third buffer, followed by incubation and centrifugation (S40), further mixing the result with a fourth buffer, followed by centrifugation (S50), further mixing the sample with isopropanol and loading the result into a column (S60), washing the sample with a fifth buffer (S70), washing the sample with a sixth buffer (S80), and washing the sample with a seventh buffer (S90).

A sample is first prepared. In an exemplary embodiment, the sample may be derived from a plant, more specifically, leaves, fruits, stems and/or roots of a plant. For example, the plant may be a tough plant rich in polyphenols and polysaccharides, such as pear, banana, strawberry, mango, grape, tomato, peach, *Arabidopsis thaliana* or pineapple. As described above, in accordance with the conventional nucleic acid extraction method, polyphenols and/or polysaccharides function as inhibitors and thus it is extremely difficult to extract high-quality nucleic acids from tough plants. However, the buffer compositions described above and the nucleic acid extraction method described later can provide high-quality nucleic acid extraction, increase reliability and accuracy of diagnosis results, and remarkably shorten time required for diagnosis.

Then, the sample is mixed with a first buffer (S10). The first buffer is mixed in an amount of about 350 µl to about 450 pl, or about 360 µl to about 440 pl, or about 370 µl to about 430 pl, or about 380 µl to about 420 pl, or about 390 µl to about 410 pl, based on 100 mg of the sample. Since the first buffer has been described above, duplicate description will be omitted.

Then, the sample is further mixed with a reducing agent in a tube (S20). The reducing agent may contain β-mercaptoethanol (99% solution), 0.5M TCEP-HCl or 1M dithiothreitol (DTT). The volume ratio of the first buffer to the reducing agent used may be about 1:0.07 to about 1:0.16, or about 1:0.08 to about 1:0.15, or about 1:0.08 to about 1:0.13. For example, the reducing agent may be mixed in an amount of about 30 µl to about 70 pl, or about 40 µl to about 60 pl, or about 50 µl. The reducing agent may contribute to the inactivation of RNase. It is preferable to thoroughly mix the tube by shaking after mixing with the reducing agent.

Then, the sample is pulverized by shaking the tube containing a mixture of the sample, the first buffer, and the reducing agent. A method of pulverizing the sample is not particularly limited and a bead beating tube or a liquid nitrogen pulverization method may be used and may be appropriately selected in consideration of the state of the sample, such as moisture content.

Then, the sample is further mixed with a second buffer in the tube (S30). The volume ratio of the first buffer to the second buffer used may be about 1:1.05 to 1:1.25, or about 1:1.1 to 1:1.2. For example, the second buffer may be mixed in an amount of about 400 µl to 500 pl, or about 410 µl to 490 pl, or about 420 µl to 480 pl, or about 430 µl to 470 pl, or about 440 µl to 460 µl. Since the second buffer has been described above, duplicate description will be omitted. It is preferable to completely mix the tube by shaking after mixing with the second buffer.

Then, the mixture containing the second buffer is incubated at about 55°C to 65°C, or about 60°C for about 2 minutes to 8 minutes, or about 3 minutes to 7 minutes (primary culture) .

Through the steps of mixing with the first buffer, mixing with the reducing agent, and mixing with the second buffer and incubation, the nucleic acid may be eluted and the RNA degrading enzyme may be inactivated, but the present invention is not limited to any theory.

Then, the result is further mixed with a third buffer in the tube (S40). The volume ratio of the first buffer to the third buffer used may be about 1:0.5 to about 1:0.9, or about 1:0.6 to about 1:0.8, or about 1:0.65 to about 1:0.75. For example, the third buffer may be mixed in an amount of about 250 µl to about 350 pl, or about 260 µl to about 340 pl, or about 270 µl to about 330 pl, or about 280 µl to about 320 pl, or about 290 µl to about 310 µl. Since the third buffer has been described above, duplicate description thereof will be omitted. It is preferable to completely mix the tube by shaking after mixing with the third buffer.

Then, the mixture containing the third buffer is incubated at room temperature, for example, at about 20°C to about 30°C, for about 30 seconds to about 5 minutes, or about 1 minute to about 3 minutes (secondary incubation).

In addition, the cultured solution is centrifuged (primary centrifugation step). The centrifugation is performed at a rate of about 10,000 rpm to about 20,000 rpm, or about 11,000 rpm to about 18,000 rpm, or about 12,000 rpm to 17,000 rpm and at 0°C to about 10°C, or about 2°C to about 8°C, or about 3°C to about 7°C, or about 4°C to about 6°C for about 3 minutes to about 10 minutes, or about 4 minutes to about 7 minutes.

The proteins and polysaccharides may be at least partially removed by mixing and incubating the third buffer, but the present invention is not limited to any theory.

Then, for example, about 2.0 ml of the centrifuged supernatant (supernatant of the primary centrifugation step), is injected into another tube and mixed with a fourth buffer (S50). The volume ratio of the first buffer to the fourth buffer used may be about 1:0.8 to about 1:1.2, or about 1:0.9 to about 1:1.1. In another aspect, the volume ratio of the supernatant in the primary centrifugation step to the fourth buffer may be about 1:0.1 to about 1:0.3, or about 1:0.15 to about 1:0.25. For example, the fourth buffer is mixed in an amount of about 350 µl to about 450 pl, or about 360 µl to about 440 pl, or about 370 µl to about 430 pl, or about 380 µl to about 420 pl, or about 390 µl to about 410 µl. Since the fourth buffer has been described above, duplicate description will be omitted. It is preferable to completely mix by shaking the tube after mixing with the fourth buffer.

In addition, the mixture containing the fourth buffer is, for example, incubated at about 20°C to about 30°C for about 30 seconds to about 5 minutes, or about 1 minute to about 3 minutes (tertiary incubation step).

In addition, the cultured product is centrifuged (secondary centrifugation step). The centrifugation is performed at a rate of about 10,000 rpm to about 20,000 rpm, or about 11,000 rpm to about 18,000 rpm, or about 12,000 rpm to about 17,000 rpm and at 0°C to 10°C, or about 2°C to about 8°C, or about 3°C to about 7°C, or about 4°C to about 6°C, for about 1 minute to about 8 minutes, or about 2 minutes to about 5 minutes.

Polyphenols may be at least partially removed by mixing and incubating the fourth buffer as described above, but the present invention is not limited to any theory.

Then, about 900 µl to 1,100 µl of the centrifuged supernatant (the supernatant of the secondary centrifugation step), for example, is injected into another tube and mixed with isopropanol (S60). The amount of isopropanol that is mixed may be about 400 µl to about 600 pl, or about 450 µl to about 550 µl. For example, the mixing ratio (v/v) of the supernatant obtained by the secondary centrifugation to isopropanol may be about 1:1.5 to about 1:2.5, or about 1:1.8 to about 1:2.3, or about 1:2.0. It is preferable to mix the resulting product by thoroughly shaking after mixing with isopropanol.

Then, the isopropanol mixture is loaded into the column. For example, an isopropanol mixture may be loaded onto a spin column to initiate extraction of nucleic acids. The step of loading the mixture on the column may be divided into two or more steps. The column may be a nucleic acid adsorbent column packed with cationic metal beads, or cationic adsorbent fibers.

However, the present invention is not limited thereto, and an ethanol precipitation method, a nucleic acid precipitation method, a magnetic bead method, or the like may be used instead of recovery using a column.

Then, the result is washed with a fifth buffer (S70). The amount of the fifth buffer used for washing may be about 500 µl or more, or about 600 µl or more, or about 700 µl or more, or about 800 µl or more, or about 900 µl or more, or about 1,000 µl or more. The upper limit of the amount of the fifth buffer is not particularly limited, but may be about 1,500 µl or less. Since the fifth buffer has been described above, duplicate description thereof will be omitted.

Washing may be performed by centrifugation. Centrifugation is performed at a rate of about 10,000 rpm to about 20,000 rpm, or about 11,000 rpm to about 18,000 rpm, or about 12,000 rpm to about 17,000 rpm and at 0°C to 10°C, or about 2°C to about 8°C, or about 3°C to about 7°C, or about 4°C to about 6°C, for about 10 seconds to about 60 seconds, or about 20 seconds to about 50 seconds, or about 30 seconds to about 40 seconds.

The pigment of the sample may be removed by this step, but the present invention is not limited to any theory.

In some embodiments, after washing with the fifth buffer, DNase treatment may be further performed. Known methods may be used for the DNase treatment and treatment conditions. Genomic DNA may be removed using DNase.

Then, the result is washed with a sixth buffer (S80). The amount of the sixth buffer used for washing may be about 500 µl or more, or about 600 µl or more, or about 700 µl or more, or about 800 µl or more, or about 900 µl or more, or about 1,000 µl or more. An amount of the sixth buffer may be smaller than an amount of the fifth buffer. The upper limit of the amount of the sixth buffer is not particularly limited, but may be about 1,500 µl or less. Since the sixth buffer has been described above, duplicate description thereof will be omitted.

Washing may be performed by centrifugation. Centrifugation is performed at a rate of about 10,000 rpm to about 20,000 rpm, or about 11,000 rpm to about 18,000 rpm, or 12,000 rpm to about 17,000 rpm and at 0°C to 10°C, or about 2°C to about 8°C, or about 3°C to about 7°C, or about 4°C to about 6°C for about 10 seconds to about 60 seconds, or about 20 seconds to 50 seconds, or about 30 seconds to about 40 seconds.

In some embodiments, the method may further include allowing the sample to stand for a predetermined time before centrifugation after loading the sixth buffer. For example, centrifugation may be performed after allowing the sample to stand for about 10 seconds to about 60 seconds, or about 20 seconds to about 50 seconds, or about 30 seconds to about 40 seconds.

Through this step, the decomposed DNA and DNase may be removed and the remaining total RNA may be bound thereto again, but the present invention is not limited to any theory.

Then, the result is washed with a seventh buffer (S90). The amount of the seventh buffer used for washing may be about 500 µl or more, or about 600 µl or more, or about 700 µl or more, or about 800 µl or more, or about 900 µl or more, or about 1,000 µl or more. An amount of the seventh buffer may be smaller than an amount of the fifth buffer. The upper limit of the amount of the seventh buffer is not particularly limited, but may be about 1,500 µl or less. Since the seventh buffer has been described above, duplicate description thereof will be omitted.

Washing may be performed by centrifugation. Centrifugation is performed at a rate of about 10,000 rpm to about 20,000 rpm, or about 11,000 rpm to about 18,000 rpm, or 12,000 rpm to about 17,000 rpm and at 0°C to 10°C, or about 2°C to about 8°C, or about 3°C to about 7°C, or about 4°C to about 6°C for about 10 seconds to about 60 seconds, or about 20 seconds to about 50 seconds, or about 30 seconds to about 40 seconds.

In some embodiments, the washing with the seventh buffer may be performed multiple times. That is, the seventh buffer may be loaded, centrifugation may be performed, the seventh buffer may be loaded again and then centrifugation may be performed again, but the present invention is not limited thereto.

The remaining salt may be removed through this step, but the present invention is not limited to any theory.

Hereinafter, although not shown in the drawings, the method may further include drying the column to remove ethanol, injecting nuclease-free water or a corresponding low-salt buffer into the column and performing centrifugation to recover or extract RNA or DNA.

### Viral diagnosis

After extraction of nucleic acids according to the present invention, the presence or absence of viruses may be diagnosed. A known amplification method such as PCR, nested-PCR, RT-PCR, ICAN, UCAN, or LAMP may be used for amplification to diagnose the presence or absence of a virus.

PCR refers to a method of amplifying the corresponding DNA region by repetition of DNA synthesis reactions between DNA polymerase and two types of primers with a specific DNA region therebetween. In some cases, PCR may encompass nested-PCR and real-time PCR. When RNA is used as a starting material (reverse transcription), RT may be performed to synthesize RNA.

The nested-PCR refers to a method of performing two-step PCR using an outer primer and an inner primer. Even if a target nucleic acid is not sufficiently amplified in the first round, it may be amplified in the second round. In addition, when non-specific amplification products are obtained in the first round of PCR, these non-specific amplification products are unlikely to have sequences similar to those of the primers in the second round of PCR. Therefore, in the second round of PCR, the probability of amplifying only the fragment having the sequence of the target may increase. RT-PCR, which is a previous step of PCR, refers to a PCR method that includes performing a reverse transcription reaction using a reverse transcriptase.

The method may further include mixing a diagnostic reagent and detecting whether or not the target DNA is present in the amplified nucleic acid sample after amplifying the nucleic acid. The detection may be performed using reagents for a probe assay, hybridization assay, oligonucleotide ligation assay, PCR (polymerase chain reaction), and LCR (ligase chain reaction).

In addition, the detection of the target DNA may be performed through DNA chips, gel electrophoresis, radioactivity measurement, fluorescence/phosphorescence spectroscopy, and the like, but the present invention is not limited thereto.

The target virus for detection may be apple necrosis virus (NC040469, NC040470, NC040471, or the like), banana bunchy top virus (NC003473, NC003474, NC003475, NC003476, NC003477, NC003479, or the like), cherry leaf roll virus (NC015414, NC015415, or the like), cherry necrotic rusty mottle virus (NC002468, or the like), citrus psorosis virus (NC006314, NC006315, NC006316, or the like), citrus ringspot virus (NC003093, or the like), citrus vein enation virus (NC021564, or the like), grapevine fanleaf virus (NC003615, NC003623, or the like), peach phony virus, peach rosette mosaic virus, peach wart virus, plum line pattern virus (NC003451, NC003452, NC003453, or the like), prune dwarf virus (NC008037, NC008038, NC008039, or the like), raspberry ringspot virus (NC005266, NC005267, or the like), tomato black ring virus (NC004439, NC004440, or the like), or tomato bushy stunt virus (NC001554, or the like).

Hereinafter, the present invention will be described in more detail with reference to experimental examples.

### Example 1. Preparation of buffers

### Example 1-1. Preparation of first buffer

25 ml of 2M Tris-HCl (pH 7.0), 2.5 ml of 0.5M EDTA, 0.25 g of sodium metabisulfite (powder), and 1.0 g of PVP K30 (powder) were mixed with each other to adjust the final volume to 50 ml. The pH of the first buffer fell within the range of 7.0 to 7.4.

### Example 1-2. Preparation of second buffer

12.5 ml of 20% SDS, 2.5 ml of 0.5M EDTA, 1.93 g of sodium citrate (powder), and 1.0 ml of 1M Tris-HCl (pH 7.4) were mixed with each other to adjust a final volume to 50 ml. The final pH of the second buffer fell within the range of 7.0 to 7.4.

### Example 1-3. Preparation of third buffer

45 ml of 5M sodium chloride, 0.37 g of potassium chloride (powder), 1.93 g of sodium citrate (powder), and 1.0 g of PVP K30 (powder) were mixed with each other to adjust the final volume to 50 ml. The final pH of the third buffer fell within the range of 7.0 to 7.4.

### Example 1-4. Preparation of fourth buffer

19.1 g of guanidine hydrochloride (Gu-HCl) (powder) and 0.75 ml of 1M Tris-HCl (pH 7.4) were mixed with each other to adjust a final volume to 50 ml. The final pH of the fourth buffer fell within the range of 7.0 to 7.4.

### Example 1-5. Preparation of fifth buffer

30 ml of 99% isopropanol solution, 1.5 ml of 5M sodium chloride, and 10 µl of 99% Tween-21 solution were mixed with each other to adjust the final volume to 50 mL. The final pH of the fifth buffer fell within the range of 7.0 to 7.4.

### Example 1-6. Preparation of sixth buffer

30 ml of a 99% ethanol solution, 7.976 g of guanidine hydrochloride (Gu-HCl) (powder), and 0.75 ml of 1M Tris-HCl (pH 7.4) were mixed with each other to adjust the final volume to 50 ml. The final pH of the sixth buffer fell within the range of 7.0 to 7.4.

### Example 1-7. Preparation of seventh buffer

37.5 ml of 99% ethanol solution, 0.2 ml of 5M sodium chloride, and 0.75 ml of 1M Tris-HCl (pH 7.4) were mixed with each other to adjust the final volume to 50 mL. The final pH of the seventh buffer fell within the range of 7.0 to 7.4.

### Example 2. RNA extraction

### Example 2-1. Sample preparation and mixing with first buffer

Mango, strawberry, grape, tomato, peach, banana, and pear leaves were prepared as samples. In addition, 100 mg of the target sample, 400 µl of the first buffer, and 50 µl of 99% β-mercaptoethanol were injected into a bead beating tube (2 ml) and pulverized at 5 m/s for 60 seconds. Then, the mixture was strongly vortexed and then spun down.

### Example 2-2. Mixing with second buffer

450 µl of the second buffer was added to the result of Example 2-1 and strongly vortexed, followed by incubation at 60°C for 5 minutes.

### Example 2-3. Mixing with third Buffer

300 µl of the third buffer was added to the result of Example 2-2 and then vigorously vortexed and the mixture was incubated at room temperature for 1 minute. In addition, the cultured solution was centrifuged at 13,000 rpm at 4°C for 5 minutes.

### Example 2-4. Mixing with fourth buffer

The entire supernatant of the result of Example 2-3 was transferred to a 2 ml tube. Then, 400 µl of the fourth buffer was added thereto and strongly vortexed, followed by incubation at room temperature for 1 minute. In addition, the cultured solution was centrifuged at 4°C at 13,000 rpm for 5 minutes.

### Example 2-5. Loading isopropanol mixture and mixing with fifth buffer

900 µl of the supernatant of the result of Example 2-4 was transferred to a 2 ml tube. Then, 450 ul of isopropanol was added and vortexed vigorously.

Then, 600 µl of the result was loaded onto a spin column and centrifuged. In addition, 700 µl of the fifth buffer was added, centrifugation was performed at 4°C and at 13,000 rpm for 30 seconds and washing was performed once.

### Example 2-6. DNase treatment and mixing with sixth buffer

Subsequently, 50 µL of a DNase solution (DNase 5 to 10 units, DNase buffer) was added to the spin column membrane through which the fifth buffer has passed, followed by reaction at room temperature for 15 minutes. After completion of the reaction, 600 µl of the sixth buffer was added, and the resulting mixture was allowed to stand for 60 seconds, was centrifuged at 4°C and at 13,000 rpm for 30 seconds and then washed once.

### Example 2-7. Washing with seventh Buffer

Then, 600 µl of the seventh buffer was added, centrifugation was performed at 4°C and at 13,000 rpm for 30 seconds and washing was then performed. This was repeated once more to perform washing a total of two times.

### Example 2-8. Nucleic acid recovery and electrophoresis

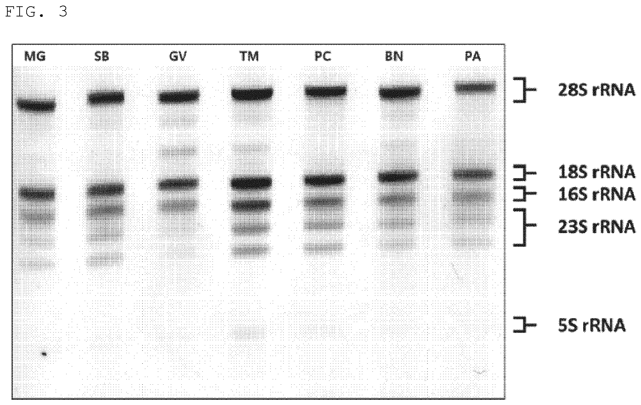

Subsequently, centrifugation was performed at 4°C and at 13,000 rpm for 2 minutes and 50 µl of RNase free water was added thereto, followed by centrifugation at 4°C and at 13,000 rpm for 30 seconds. 200 ng of the recovered RNA was electrophoresed on a Bleach gel containing 2% agarose and 0.5X TBE. The results are shown in FIG. 3.

As can be seen from FIG. 3, nucleic acids with excellent quality could be extracted from all seven samples using the buffers and extraction method according to the present invention.

### Comparative Example 1. Recovery of nucleic acid using products from other companies

### Comparative Example 1-1. QIAGEN N.V.

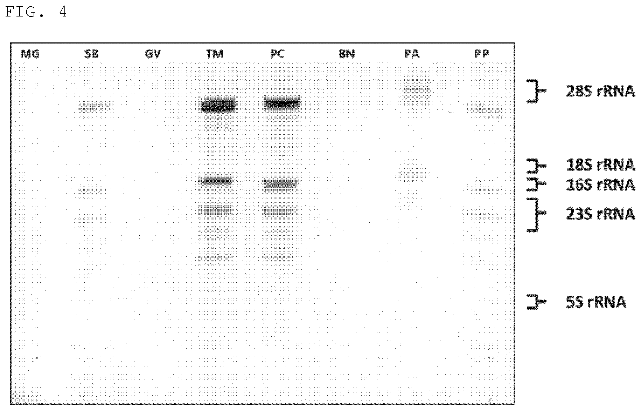

Nucleic acids were extracted from mangoes (MG), strawberries (SB), grapes (GV), tomatoes (TM), peaches (PC), bananas (BN), pear tree (PA), and pepper (PP) using a RNeasy plant mini kit from QIAGEN N.V. (hilden, Germany) and the results of electrophoresis of the extracted nucleic acids are shown in FIG. 4.

As can be seen from FIG. 4, the quality and yield of RNAs extracted from samples other than tomatoes (TM) and peach (PC) were low and the RNAs were degraded.

That is, the RNeasy plant mini kit commercially available from Qiagen N.V. is not universally applicable to various plants.

### Comparative Example 1-2. Norgen Biotek Corp.

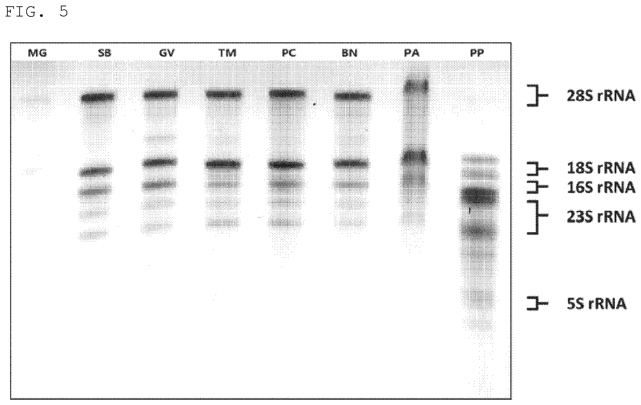

Nucleic acids were extracted from mangoes (MG), strawberries (SB), grapes (GV), tomatoes (TM), peaches (PC), bananas (BN), pear tree (PA), and pepper (PP) using a plant RNA/DNA purification kit (Cat. 24400) from Norgen Biotek Corp. (Thorold, Canada) and the results of electrophoresis of the extracted nucleic acids are shown in FIG. 5.

As can be seen from FIG. 5, nucleic acid was degraded in mangoes (MG). Products other than nucleic acids were also extracted from pear tree (PA), inducing a band attraction. In red pepper (PP), RNA with a larger molecule weight such as 28S rRNA was completely degraded.

That is, the RNeasy plant mini kit commercially available from Norgen Biotech is not universally applicable to various plants.

### Comparative Example 2. Recovery of nucleic acid from pepper leaves

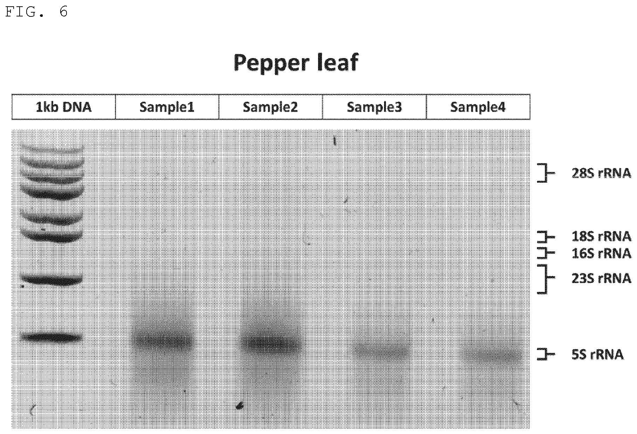

Nucleic acid was extracted and electrophoresis was performed in the same manner as in Example 2, except that pepper leaves were used as samples. In addition, the results are shown in FIG. 6.

As can be seen from FIG. 6, although nucleic acid extraction was performed in the same manner as in Example 2, the quality and yield of the extracted RNA was low and the RNA was degraded.

### Comparative Example 3. Variations in buffer concentrations and order

### Comparative Example 3-1. Without Example 2-2 (Sample 1)

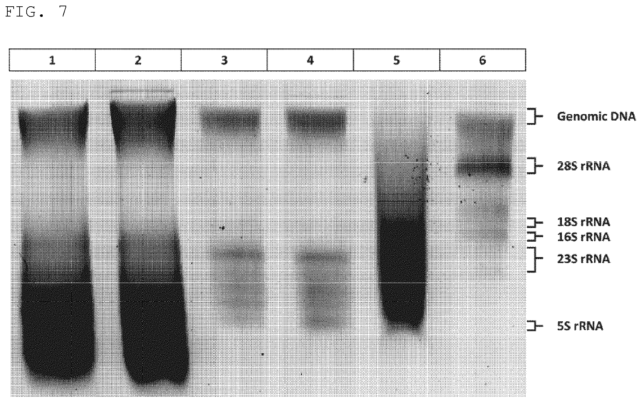

Nucleic acid recovery and electrophoresis were performed in the same manner as in Example 2, except that a banana sample was used and Example 2-3 was performed, immediately without mixing with the second buffer, and the results are shown in Sample 1 of FIG. 7.

### Comparative Example 3-2. Change of order of Example 2-2 and Example 2-3 (Sample 1)

Nucleic acid recovery and electrophoresis were performed in the same manner as in Example 2, except that a banana sample was used and the order of Example 2-2 and Example 2-3 was changed, and the results are shown in Sample 2 of FIG. 7.

### Comparative Example 3-3. Change of composition of the third buffer (Sample 3)

Nucleic acid recovery and electrophoresis were performed in the same manner as in Example 2, except that a banana sample was used and 3M NaOAc was used instead of 4.5M sodium chloride in Example 2-3, and the results are shown in Sample 3 of FIG. 7.

For reference, Sample 4 is the result of an experiment unrelated to the present invention.

### Comparative Example 3-4. Homogenization with third buffer (Sample 5)

Nucleic acid recovery and electrophoresis were performed in the same manner as in Example 2, except that a banana sample was used, the third buffer was used instead of the first buffer in Example 2-1, and the first buffer was used instead of the third buffer in Example 2-3, and the results are shown in Sample 5 in FIG. 7.

For reference, Sample 6 is the result of an experiment unrelated to the present invention.

As can be seen from FIG. 7, RNA is completely degraded in all samples.

### Comparative Example 4. Change in composition of buffer

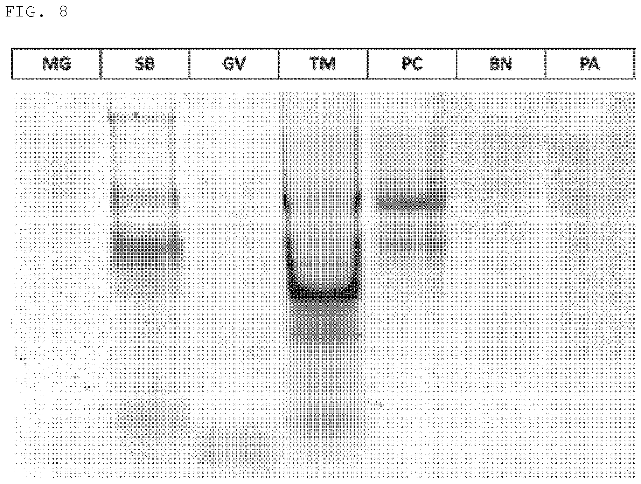

Nucleic acid recovery and electrophoresis were performed in the same manner as in Example 2, except that guanidine thiocyanate was used as the fourth buffer instead of guanidine hydrochloride in Example 2-4, and the results are shown in FIG. 8.

As can be seen from FIG. 8, RNA was degraded in all samples and the RNA yield was low.

### Comparative Example 5. Without heating

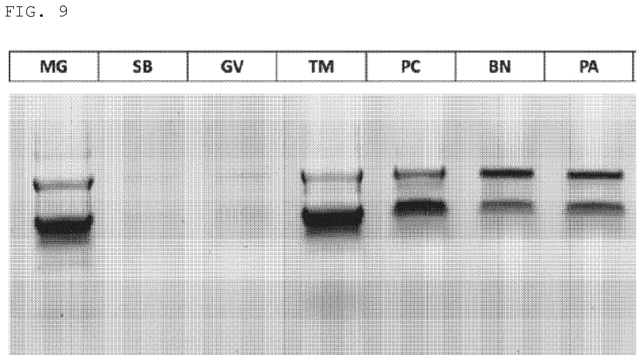

Nucleic acid recovery and electrophoresis were performed in the same manner as in Example 2, except that heating at 60°C was not performed in Example 2-2, and the results are shown in FIG. 9.

As can be seen from FIG. 9, the amount of RNA extracted from strawberries (SB) and grapes (GV) was significantly reduced.

### Experimental Example 1. Measurement of RNA purity and extraction amount

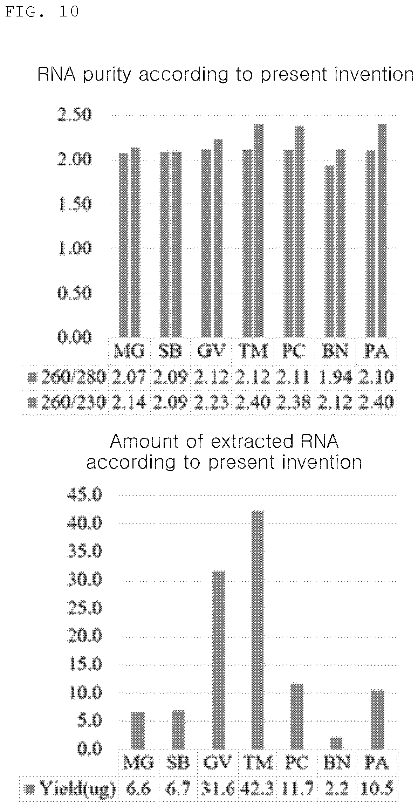

The purity and extraction amounts of nucleic acids were measured in accordance with the methods of Example 2, Comparative Example 1-1, and Comparative Example 1-2. In addition, the results are shown in FIGS. 10 to 12.

RNA and DNA strongly absorb the UV wavelength at 260 nm, proteins and phenols strongly absorb the wavelength at 280 nm, and salts, carbohydrates and phenols strongly absorb the wavelength at 230 nm. Absorbance of the extracted RNA may be measured with a UV spectrometer based on pure water. A value obtained by dividing the absorbance at 260 nm by the absorbance at 230 nm or the absorbance at 280 nm is defined as A260/A230 or A260/A280, respectively.

RNA having an absorbance at 260 nm that is about twice higher than that of the absorbance at 230 nm or 280 nm may be considered pure and the absorbance at 260 nm decreases as the contamination of the sample increases. For example, when RNA is contaminated with a guanidine salt or phenol, the absorbance at 230 nm thereof increases. In particular, when contamination with phenol increases, the absorbance at 260 nm increases, which may be overestimated compared to the actual amount of extracted RNA. That is, when high-purity RNA is extracted, the value of A260/A230 or A260/A280 is between 2.0 and 2.2.

As can be seen from FIG. 10, higher or at least similar quality and recovery rates compared to commercial kits, which are control groups, were observed in all of mangoes (MG), strawberries (SB), grapes (GV), tomatoes (TM), peaches (PC), bananas (BN) and pears (PA). In particular, it was found that the absorbance ratio of all samples was 2.0 or higher, which means that high-quality nucleic acid can be extracted.

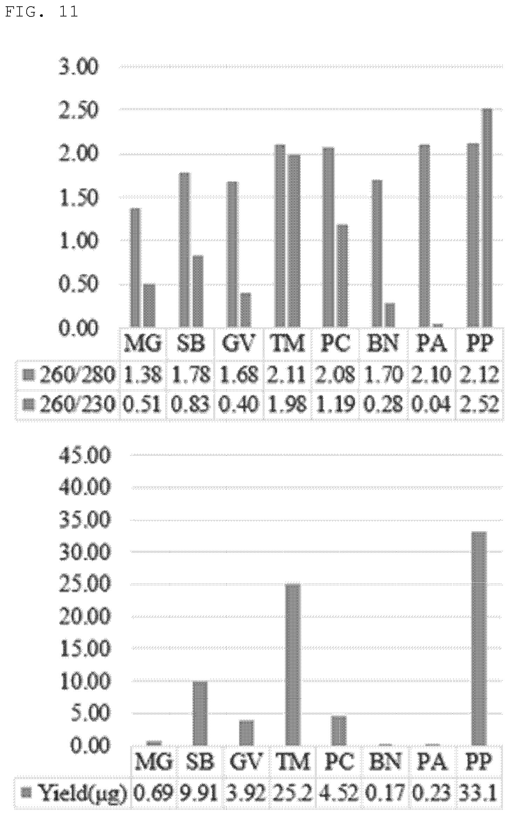

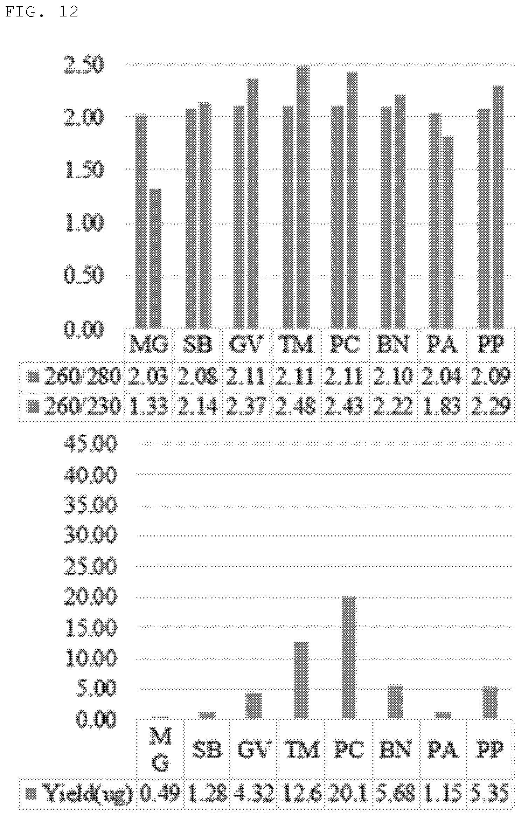

On the other hand, as can be seen from FIGS. 11 and 12, the kit from Qiagen Inc. could successfully complete extraction of RNA from tomatoes, peaches, and strawberries, but exhibited low quality and recovery of RNAs from mangoes, grapes, bananas, and pears. In addition, the kit from Norgen exhibited low quality and recovery from mango and red pepper.

### Experimental Example 2. RNA amplification curve and melting curve

In order to determine whether or not the RNA extracted in Example 2 is suitable for downstream analysis such as qPCR, 1 ug of total RNA was reverse transcribed to produce cDNA, and the house-keeping gene of each sample was amplified by PCR.

cDNA synthesis was performed by adding 1 µg of total RNA using AccuPower^{®} CycleScript^{™} RT PreMix (dN6) (Bioneer, Daejeon, South Korea) in accordance with the manufacturer's protocol. The synthesized cDNA was diluted 10x and used as a template.

In addition, genes expressed at low levels were selected through literature search. Genes and primer sequences thereof used in real-time PCR analysis are summarized in Table 1 below.

**[Table 1]**

| Plant names | Forward primers | Reverse primers | Referenc es |
|---|---|---|---|
| Mango | >ACT7 CGTTCTGTCCCTCTATGCCA | >ACT7 AGATCACGGCCAGCAAGATC | (Islas-Osuna et al., 2019) |
| | >GAPDH GTGGCTGTTAACGATCCCTT | >GAPDH GTGACTGGCTTCTCATCGAA | |
| Strawber ry | >18S | >18S | (Zhang et al., 2018) |
| | TGTGAAACTGCGAATGGCTCA TTAA | GAAGTCGGGATTTGTTGCACG TATT | |
| | >GAPDH GAGTCTACTGGAGTGTTCA | >GAPDH CTTGTATTCGTGCTCATTCA | |
| | >ACTIN2 GCTAATCGTGAGAAGATGAC | >ACTIN2 AGCACAATACCAGTAGTACG | |
| Grapevin e | >VvCYP ACAGCCAAGACCTCGTG | >VvCYP GCCTTCACTGACCACAAC | (Borges et al., 2014) |
| | >VvACT GACTACCTACAACTCCATCAT | >VvACT TCATTCTGTCAGCAATACCA | |
| Tomato | >EF1a | >EF1a CCAGTAGGGCCAAAGGTCACA | (Müller et al., 2015) |
| | GGTGGTTTTGAAGCTGGTATC TCC | | |
| | | >GAPDH | |
| | >GAPDH | CTTCCTCCAATAGCAGAGGTT T | |
| | CTGCTCTCTCAGTAGCCAACA C | | |
| | | >CAC | |
| | >CAC CCTCCGTTGTGATGTAACTGG | ATTGGTGGAAAGTAACATCAT CG | |
| Peach | >Got1 GT T GGCAGT TGGAAGCACAGC | >Got1 | (Bastias et al., 2020) |
| | | AAATAAGGGCGCAGACCTCAC AT | |
| | >GAPDH | | |
| | TTTGAAGGGTGGAGCGAAGAA GG | >GAPDH | |
| | | ATGGAGTGAACGGTGGTCATC AG | |
| Banana | >ACT1pp2 GAGCGGAAGTACAGTGTCTGG | >ACT1pp2 AGAAGCACTTCCTGTGGACAA | (Podevin et al., 2012, Rego et al., 2019) |
| | >EF1a CGGAGCGTGAAAGAGGAAT | >EF1a ACCAGCTTCAAAACCACCAG | |
| | >GAPDH CATCAAGCAAGGACTGGAGAG | >GAPDH AAGCAGGGAGAACTTTTCCAA | |
| Pear | >ACTIN2 CTCCCAGGGCTGTGTTTCCTA | >ACTIN2 CTCCATGTCATCCCAGTTGCT | (Liu et al., 2018) |
| | >Histone H3 GTCAAGAAGCCCCACAGATAC | >Histone H3 | |
| | | CTGGAAACGCAGATCAGTCTT G | |

Realtime PCR was performed from cDNA prepared using primers specific to the reference genes. Real-time PCR was performed using qTower3 (Analytik Jena, Jena, Germany) equipment. A single reaction was performed in triplicate using 10 µl of 2X SFC green qPCR master mix (SFC Co., Ltd., Chungbuk, South Korea) as a PCR mix, 5 µl of a template, 2 µl of a primer mix (10 uM of each of forward and reverse), and 3 µl of RNase free water, heating was performed at 95°C for 5 min for heat activation and pre-denaturation, and 45 cycles at 95°C for 15 seconds, at 58°C for 15 seconds, and at 72°C for 20 seconds (FAM fluorescence detection) were repeated.

In order, ACT7 and GAPDH were selected from mango (Islas-Osuna *et al.,* 2019), 18S rRNA, GAPDH, and Actin2 were selected from strawberries (Zhang *et al.,* 2018), VvCYP and VvACT were selected from grapevines (Borges *et al.,* 2014), GAPDH, CAC, and EF1a were selected from tomatoes (Müller *et al.,* 2015), GAPDH and Got1 were selected from peaches (Bastias *et al.,* 2020), ACT1pp2, GAPDH and EF1a were selected from bananas (Podevin *et al.,* 2012, Rego *et al.,* 2019), and H3 and Actin2 were selected from pears (Liu *et al.,* 2018). (Cheng *et al.,* 2017, Müller *et al.,* 2015).

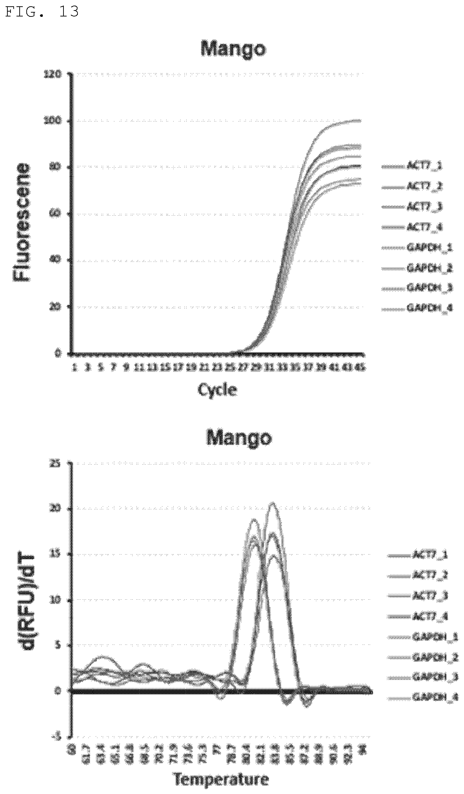

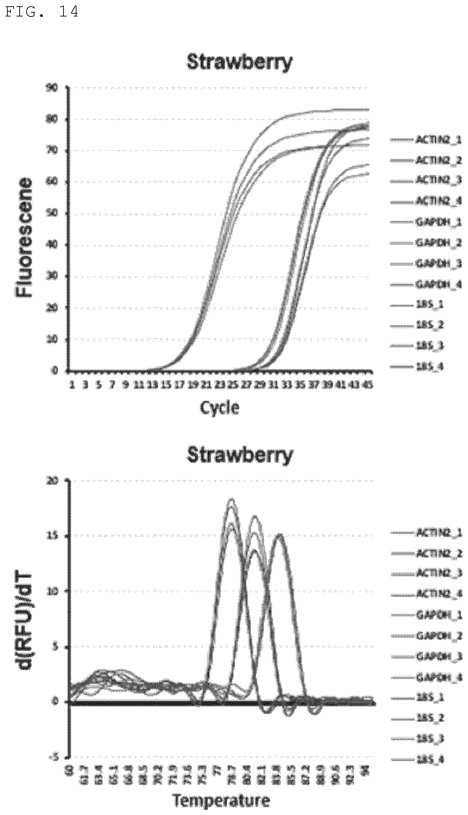

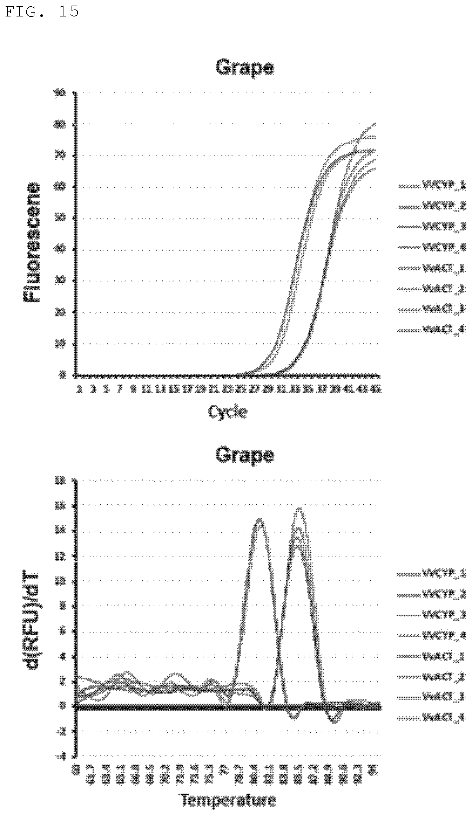

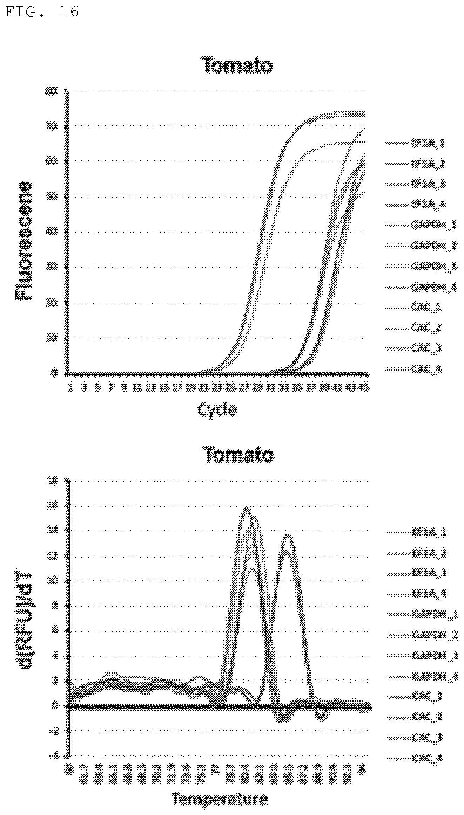

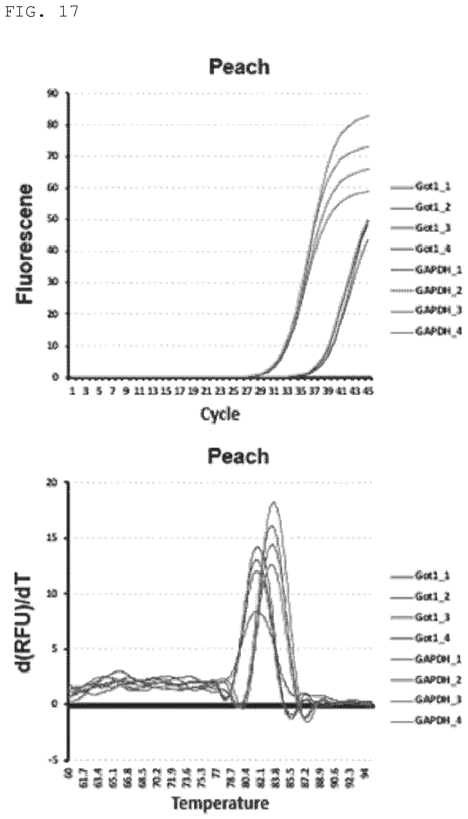

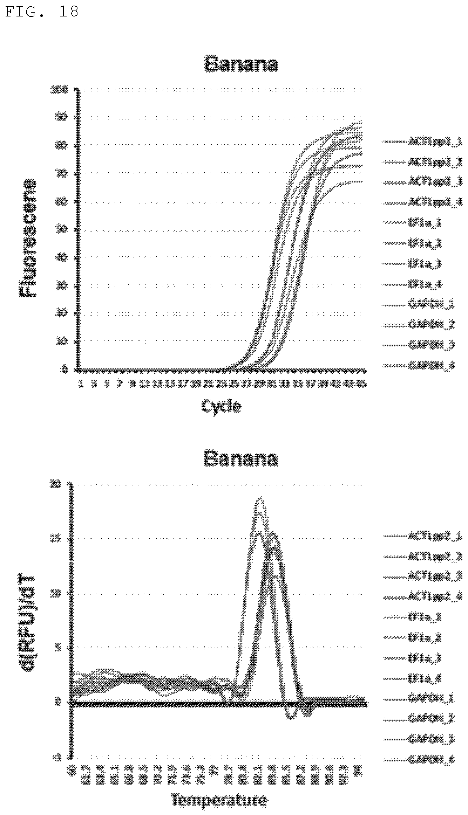

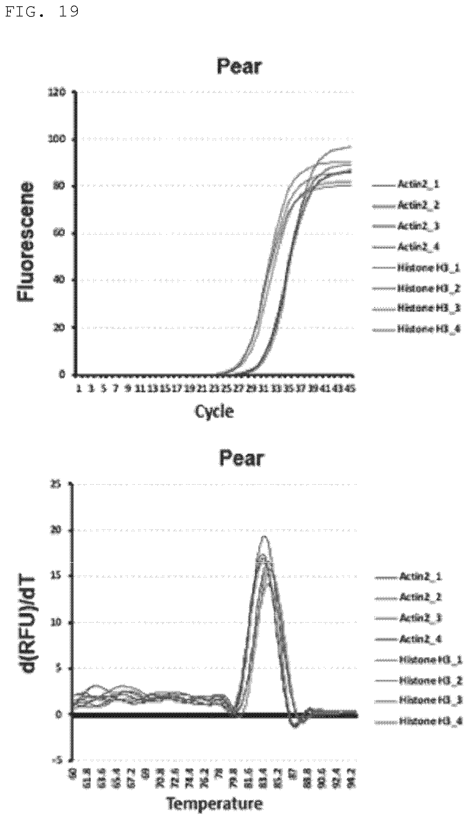

In addition, the results are shown in FIGS. 13 to 19. As can be seen from FIGS. 13 to 19, each gene was amplified, the fluorescence equal to or higher than the baseline threshold was measured, and a typical pattern, an initiation phase, an exponential phase, and a linear phase observed in a normal real-time PCR amplification process were observed.

After completion of the PCR, FAM fluorescence was detected by holding at 0.5°C for 15 seconds from 65°C to 95°C after heating at 95°C for 1 minute for melting curve analysis (Ririe et al., 1997). The result of melting curve analysis shows that the melting temperature of each PCR product ranged from the minimum of 78.62°C to the maximum of 85.48°C and the melting curve also formed a single peak without any peak suspected of contamination of gDNA or formation of non-specific products.

Although embodiments of the present invention have been described in detail, they are merely provided for illustration of the present invention and should not be construed as limiting the scope of the present invention. Those skilled in the art will appreciate that various modifications and applications are possible, without departing from the scope and spirit of the embodiments of the present invention.

Therefore, it should be understood that the scope of the present invention includes alterations, equivalents, or substitutes of the technical ideas exemplified above. For example, each element specifically disclosed in the embodiment of the present invention may be implemented in modified forms. Also, differences related to such modifications and applications should be construed as falling within the scope of the present invention defined in the appended claims.

This work was supported by Korea Institute of Planning and Evaluation for Technology in Food, Agriculture and Forestry(IPET) through Technology Commercialization Support Program, funded by Ministry of Agriculture, Food and Rural Affairs(MAFRA) (No. 821044-3).

## Claims

1. A method of extracting nucleic acids from a sample, comprising the following steps:
mixing the sample with a first buffer comprising Tris-HCl (tris(hydroxymethyl)aminomethane-HCl);
mixing the sample with a second buffer comprising sodium dodecyl sulfate (SDS);
mixing the sample with a third buffer comprising sodium chloride (NaCl); and
mixing the sample with a fourth buffer comprising guanidine hydrochloride (Gu-HCl).

2. The method according to claim 1, wherein the steps are performed sequentially.

3. The method according to claim 2, wherein the first buffer comprises 0.8M to 1.2M Tris-HCl, and
the first buffer further comprises:
20 mM to 30 mM ethylenediaminetetraacetic acid (EDTA);
0.3% (w/v) to 0.7% (w/v) of sodium metabisulfite, sodium sulfite, sodium acid sulfite, or sodium sulfate; and
1.8% (w/v) to 2.2% (w/v) of polyvinylpyrrolidone, polyvinylpolypyrrolidone, or polyethylene glycol.

4. The method according to claim 2, wherein the second buffer comprises 3% (w/v) to 8% (w/v) of SDS, and
the second buffer further comprises:
20 mM to 30 mM EDTA;
120 mM to 180 mM sodium citrate; and
15 mM to 25 mM Tris-HCl.

5. The method according to claim 2, wherein the third buffer comprises 4.2M to 4.8M sodium chloride, and
the third buffer further comprises:
80 mM to 120 mM potassium chloride, calcium chloride, ferric chloride, ferrous chloride, ammonium aluminum or ammonium sulfate; and
1.8% (w/v) to 2.2% (w/v) of polyvinylpyrrolidone, polyvinylpolypyrrolidone, or polyethylene glycol.

6. The method according to claim 2, wherein the fourth buffer comprises 3.5M to 4.5M guanidine hydrochloride, and
the fourth buffer further comprises 10 mM to 20 mM Tris-HCl.

7. The method according to claim 2, wherein a ratio of used volume of the first buffer to the second buffer is 1:1.1 to 1:1.2,
a ratio of used volume of the first buffer to the third buffer is 1:0.6 to 1:0.8,
a ratio of used volume of the first buffer to the fourth buffer is 1:0.9 to 1:1.1, and
the first buffer is used in an amount of 350 µl to 450 µl.

8. The method according to claim 1, wherein the step of mixing the sample with the first buffer comprises:
mixing the first buffer, the sample, and 40 µl to 60 µl of a reducing agent, and
pulverizing the sample,
wherein the reducing agent comprises β-mercaptoethanol, 0.3M to 0.7M TCEP-HCl, or 0.8M to 1.2M dithiothreitol (DTT), and
a ratio of used volume of the first buffer to the reducing agent is 1:0.08 to 1:0.15.

9. The method according to claim 8, further comprising the following steps:
primary incubation of incubating the mixture at 55°C to 65°C for 2 to 8 minutes after mixing with the second buffer and before mixing with the third buffer;
secondary incubation of incubating the mixture for 30 seconds to 5 minutes after mixing with the third buffer and before mixing with the fourth buffer;
primary centrifugation of centrifuging the cultured product after the secondary incubation and before mixing with the fourth buffer;
tertiary incubation comprising incubating the mixture after mixing with the fourth buffer;
secondary centrifugation of centrifuging the cultured product; and
mixing the supernatant obtained from the secondary centrifugation with isopropanol and loading the mixture onto a column,
wherein the mixing with the fourth buffer comprises mixing the supernatant obtained from the primary centrifugation with the fourth buffer.

10. The method according to claim 9, further comprising the following steps sequentially:
primary washing of washing the column with a fifth buffer containing isopropanol and sodium chloride;
secondary washing of washing the column with a sixth buffer containing ethanol, Tris-HCl, and guanidine hydrochloride;
tertiary washing of washing the column with a seventh buffer containing ethanol and Tris-HCl; and
recovering nucleic acids.

11. The method according to claim 1, wherein the sample is derived from fruit crops including pears, bananas, strawberries, mangoes, grapes, tomatoes, peaches, *Arabidopsis thaliana,* or pineapples.

12. A kit for extracting nucleic acids, the kit comprising a plurality of buffers comprising a first buffer, a second buffer, a third buffer, and a fourth buffer,
wherein the kit is configured to mix the sample with the first buffer and a reducing agent, to pulverize the sample, to mix the sample with the second to fourth buffers, to mix the sample with isopropanol, to load the sample on a column, and to wash the sample with at least one wash buffer.
